# FASCICULE DE BREVET EUROPEEN

(11) **EP 0 562 892 B1**
(45) Date de publication et mention de la délivrance du brevet: **23.04.1997**
(21) Numéro de dépôt: 93400441.7
(22) Date de dépôt: 19.02.1993
(51) Int. Cl.: G01N 33/20

(54) **Procédé d'évaluation de la propreté inclusionnaire d'un produit métallurgique**
Verfahren zur Bewertung der Anwesenheit von Einschlüssen in einem metallurgischen Produkt
Process for evaluating the presence of inclusions in a metallurgical product

(30) Priorité: 27.03.1992 FR 9203748
(43) Date de publication de la demande: 29.09.1993
(73) Titulaire: SOLLAC, F-92800 Puteaux (FR)
(72) Inventeur: Debiesme, Bernard, F-59240 Dunkerque (FR); Amoris, Eric, F-59240 Dunkerque (FR); Perdrix, Christian, F-59240 Dunkerque (FR); Poissonnet, Isabelle, F-59470 Wormhout (FR); Margo-Marette, Hélène, F-78100 Saint Germain en Laye (FR)
(74) Mandataire: Bouget, Lucien

(56) Documents cités:
- US-A- 2 595 295
- US-A- 4 067 751
- JAPANESE INDUSTRIAL STANDARD vol. JIS G, no. 0555, 1977, TOKYO, JAPAN pages 1 - 4 'MICROSCOPIC TESTING METHOD FOR THE NON-METALLIC INCLUSIONS IN STEEL'
- PATENT ABSTRACTS OF JAPAN vol. 9, no. 276 (P-402)(1999) 2 Novembre 1985
- PATENT ABSTRACTS OF JAPAN vol. 8, no. 286 (P-324)(1723) 27 Décembre 1984

## Description

L'invention concerne un procédé d'évaluation de la propreté inclusionnaire d'un produit métallurgique.

Les produits métallurgiques, et en particulier les produits sidérurgiques constitués d'acier peuvent renfermer de petites particules d'oxydes ou de sulfures appelées inclusions. La présence plus ou moins grande de ces inclusions définit ce qu'on appelle la propreté inclusionnaire : moins il y a d'inclusions, plus l'acier est dit propre.

Il existe de nombreuses techniques pour évaluer la propreté inclusionnaire :
- dosage chimique de l'oxygène dans l'acier,
- examens métallographiques,
- attaque par un acide et comptage de porosités,
- dissolution électrolytique et pesée des résidus.

A cet égard, on peut signaler le document *"Japanese Industrial Standard,* vol. JIS G, no. 0555, 1977, Tokyo, Japan, pages 1-4" et la demande JP-A-60 120 252 qui présentent respectivement une méthode comportant un examen microscopique et une méthode comprenant l'application d'un papier imprégné d'une solution de carboxylate d'aurine.

Mais ces méthodes soit manquent de sensibilité, soit nécessitent d'examiner des échantillons de taille importante et de ce fait sont très longues à mettre en oeuvre.

Le but de la présente invention est de proposer un procédé d'évaluation de la propreté inclusionnaire d'un produit métallurgique par comptage d'inclusions qui soit sensible et rapide.

A cet effet, l'invention a pour objet un procédé d'évaluation de la propreté inclusionnaire d'un produit métallurgique par comptage d'inclusions à l'intérieur du produit selon lequel :
- on prélève un échantillon du produit métallurgique et on usine une éprouvette à partir de cet échantillon, par enlèvement de matière, de façon à créer des surfaces externes de l'éprouvette, d'aire déterminée, à différentes profondeurs sous les surfaces externes initiales du produit métallurgique,
- on immerge l'éprouvette dans un bain contenant des ions hydrogène et un inhibiteur de recombinaison d'hydrogène ou un promoteur d'absorption d'hydrogène, pendant une durée suffisante pour obtenir une pénétration significative d'hydrogène à l'intérieur de l'éprouvette,
- on examine les surfaces externes de l'éprouvette, de manière à détecter et à compter, pour chacune des surfaces externes le nombre de cloques formées par soulèvement d'une couche superficielle du produit sous l'effet d'hydrogène sous pression accumulé au niveau de chacune des inclusions situées sous la couche superficielle, au voisinage de la surface externe.

Le bain dans lequel est immergé l'éprouvette peut être constitué par une solution acide non oxydante et par exemple par de l'eau saturée de chlorure de sodium, dont le pH est maintenu à 3 par de l'acide acétique, qui est saturée en sulfure d'hydrogène H₂S et dont la température est voisine de l'ambiante ; ce milieu est réalisé conformément aux recommandations TM 01-77 et TM 02-84 de la Nationale Association for Corrosion Engineers (NACE) à l'exception du paragraphe 5-3 de la recommandation TM 02-84 relative au temps de maintien de l'éprouvette dans le milieu.

Dans ce cas, on immerge l'éprouvette dans ledit milieu pendant une durée qui est de préférence de quatre heures environ.

Lorsque le produit sidérurgique est une tôle ou une plaque, l'échantillon est un parallélépipède et l'éprouvette est obtenue en usinant ledit parallélépipède de façon à réaliser deux séries de gradins constitués de faces parallèles situées à des profondeurs croissantes sous la surface de la tôle, une première série de gradins partant de la surface supérieure de la tôle, une deuxième série de gradins partant de la surface inférieure de la tôle et les deux séries de gradins étant disposées de telle sorte que chaque face d'une série de gradins se trouve en vis-à-vis d'une face de l'autre série de gradins et que la distance entre deux faces opposées soit la même pour toutes les faces.

Les aires des faces des séries de gradins sont de préférence toutes égales entre elles.

Avec une telle éprouvette, on compte, sur chaque face des séries de gradins, les cloques dues au soulèvement d'une couche superficielle de l'acier par l'hydrogène accumulé au niveau de chacune des inclusions situées sous la couche superficielle de la face afin d'obtenir une évaluation de la propreté inclusionnaire à différentes profondeurs sous la surface de la tôle.

Ce procédé permet d'obtenir très rapidement une évaluation de la propreté inclusionnaire en ayant un très bon échantillonnage puisque les aires des surfaces soumises au milieu agressif contenant de l'hydrogène peuvent être importantes.

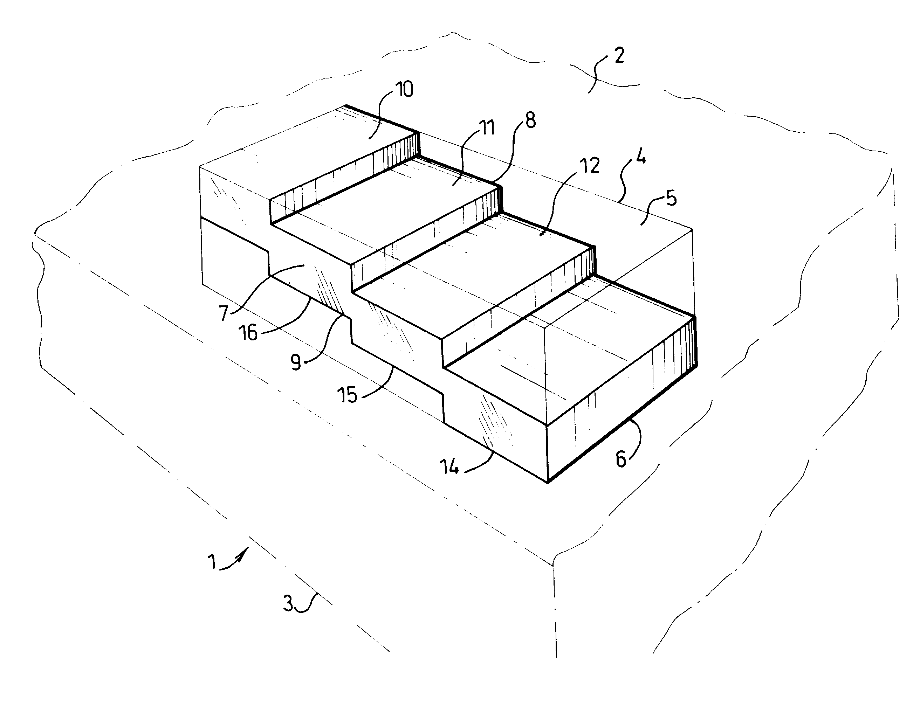

L'invention va maintenant être décrite plus en détail en regard de l'unique figure annexée qui représente en perspective éclatée une partie d'une tôle et une éprouvette usinée dans cette tôle.

Pour évaluer la propreté inclusionnaire d'une tôle 1 possédant une face supérieure 2 et une face inférieure 3, on prélève un échantillon parallélépipédique 4 dont deux faces externes opposées 5 et 6 correspondent aux surfaces externes 2 et 3 de la tôle.

Dans cet échantillon 4 on usine par enlèvement de matière, une éprouvette 7 en réalisant une série de gradins supérieure 8 et une série de gradins inférieure 9. Chaque série de gradins est constituée de faces externes 10, 11, 12, ... et 14, 15, 16 ... parallèles aux faces 5 et 6 correspondant aux surfaces externes initiales 2 et 3 de la tôle et situées à des profondeurs croissantes sous ces faces.

Ainsi la face 10 est confondue avec la face 5 ; la face 11 est à une certaine profondeur sous la face 5 par exemple 0,5 mm ; la face 12 est à une profondeur plus importante que la face 11, par exemple 1 mm. De même, la face 14 coincide avec la face 6 de l'échantillon, la face 15 est à une certaine profondeur sous la face 6, par exemple 0,5 mm ; la face 15 est à une profondeur plus importante par exemple 1 mm sous la face 6. Toutes les faces 10, 11, 12 ... 14, 15, 16 ... ont la même aire. Les faces de la série de gradins supérieure 8 sont en vis-à-vis des faces de la série de gradins inférieure 9 et ces faces sont parallèles entre elles. L'écart de profondeur entre deux faces successives des deux séries de gradins est ajusté de telle sorte que l'épaisseur de métal comprise entre deux faces en vis-à-vis soit constante dans toute l'éprouvette. Ainsi l'épaisseur de métal compris entre les faces 12 et 15 est la même que l'épaisseur de métal comprise entre les faces 11 et 16.

A titre d'exemple, pour une tôle de 10 mm d'épaisseur, chaque face des gradins mesure 100 mm de long sur 35 mm de large et la différence de profondeur entre deux faces successives est de 0,5 mm. Si chaque gradin comporte 11 faces, l'épaisseur de métal entre deux faces en vis-à-vis est de 5 mm.

Les faces qui correspondent à la surface de la tôle sont polies, les autres faces sont rectifiées.

Après usinage, on plonge l'éprouvette dans un bain constitué d'eau saturée en chlorure de sodium, dont le pH est fixé à 3 par un ajout d'acide acétique, saturé en sulfure d'hydrogène H₂S et maintenu à la température ambiante selon les recommandations TM 01-77 et TM 02-84 pour l'essai HIC définies par la National Association of Corrosion Engineers (NACE) à l'exception de l'alinéa 5.3 du TM 02-84.

On maintient l'éprouvette dans le bain pendant quatre heures. Les ions hydrogène du bain en contact avec la surface externe de l'éprouvette diffusent à l'intérieur de l'acier et se rassemblent de manière préférentielle sur les inclusions. Par recombinaison des atomes d'hydrogène, il se forme des molécules d'hydrogène gazeux qui s'accumulent au niveau de chacune des inclusions de manière que la pression de l'hydrogène gazeux augmente au cours du temps. Pour chacune des faces en gradins de la surface externe de l'éprouvette, il se produit une déformation en forme de cloque, au-dessus des inclusions situées au voisinage de la face et séparées de celle-ci par une mince couche superficielle d'acier dont l'épaisseur peut aller jusqu'à 0,5 mm. Les cloques sont produites par soulèvement de la couche superficielle par l'hydrogène sous pression présent au niveau des inclusions.

De ce fait, lorsqu'on retire l'éprouvette du bain sa surface externe comporte des cloques sur chacune des faces en gradins ; on compte sur chaque face le nombre de cloques. L'examen des faces de l'éprouvette permettant de réaliser le comptage des cloques peut être effectué en utilisant des moyens classiques dans le domaine de la métallographie. Pour chaque face, le nombre de cloques est représentatif de la propreté inclusionnaire de la tôle à une profondeur sous la peau ou surface, égale à la profondeur d'usinage de la face.

Ce procédé permet d'évaluer la propreté inclusionnaire de l'acier. En effet, comme expliqué plus haut, lorsque l'éprouvette est plongée dans le bain actif, de l'hydrogène pénètre dans le métal, vient se concentrer autour des inclusions et crée autour de celles-ci une surpression qui provoque l'apparition de cloques. Ainsi, à chaque cloque correspond au moins une inclusion et le comptage du nombre de cloques est un bon indicateur du nombre d'inclusions qui sont proches de la surface de chaque face.

Ce procédé peut être utilisé également pour caractériser la propreté inclusionnaire de produits longs par exemple de fils ou de barres dans lesquels on usine des éprouvettes en gradins cylindriques. Cependant, dans ce cas, l'épaisseur de métal à chaque gradin n'est pas constante.

Le procédé peut aussi être utilisé pour évaluer la sensibilité des aciers IFS (Interstitial free steels) au défaut de boursoufflures qui apparaît parfois après recuit sous hydrogène.

On remarquera que pour faire cet essai il suffit de faire pénétrer de l'hydrogène dans le métal ; pour celà on peut utiliser soit l'immersion simple soit le chargement cathodique en utilisant un bain acide non oxydant auquel on a ajouté un inhibiteur de recombinaison d'hydrogène ou encore un promoteur d'absorption d'hydrogène.

Dans le cas d'un chargement cathodique de l'éprouvette, celle-ci est portée à un certain potentiel électrique et constitue la cathode d'une installation d'électrolyse du bain. Le transport des ions hydrogène dans le bain et le chargement de l'éprouvette en hydrogène sont assurés par le passage d'un courant électrique dans le bain.

Outre le milieu défini par les recommandations du NACE, on peut utiliser les milieux sulfuriques auxquels est ajouté de l'arséniate de sodium et d'autres milieux connus de l'homme du métier.

## Revendications

1. Procédé d'évaluation de la propreté inclusionnaire d'un produit métallurgique (1) par comptage d'inclusions à l'intérieur du produit (1), caractérisé en ce que :
- on prélève un échantillon (4) du produit métallurgique (1) et on usine une éprouvette (7) à partir de cet échantillon, par enlèvement de matière, de façon à créer des surfaces externes (11, 12, 15, 16) de l'éprouvette, d'aire déterminée, à différentes profondeurs sous les surfaces externes initiales (2, 3) du produit métallurgique (1),
- on immerge l'éprouvette (7) dans un bain contenant des ions hydrogène et un inhibiteur de recombinaison d'hydrogène ou un promoteur d'absorption d'hydrogène, pendant une durée suffisante pour obtenir une pénétration significative d'hydrogène à l'intérieur de l'éprouvette (7),
- on examine les surfaces externes (10, 11, 12, 14, 15, 16) de l'éprouvette (7), de manière à détecter et à compter, pour chacune des surfaces externes (10, 11, 12, 14, 15, 16) le nombre de cloques formées par soulèvement d'une couche superficielle du produit (1) sous l'effet d'hydrogène sous pression accumulé au niveau de chacune des inclusions situées sous la couche superficielle, au voisinage de la surface externe.

2. Procédé suivant la revendication 1, caractérisé en ce que le bain est constitué par une solution acide non oxydante.

3. Procédé selon la revendication 2, caractérisé en ce que le bain est constitué d'eau saturée en chlorure de sodium, dont le pH est maintenu à 3 par de l'acide acétique, qui est saturée en sulfure d'hydrogène H₂S et dont la température est voisine de l'ambiante.

4. Procédé suivant la revendication 1, caractérisé en ce que l'éprouvette (7) est portée à un potentiel cathodique pendant l'immersion dans le bain.

5. Procédé selon la revendication 3, caractérisé en ce qu'on immerge l'éprouvette (7) dans le bain pendant quatre heures environ.

6. Procédé selon l'une quelconque des revendications 1 à 5 caractérisé en ce que, lorsque le produit sidérurgique (1) est une plaque ou une tôle, l'échantillon (4) est un parallélépipède et l'éprouvette (7) est obtenue en usinant ledit parallélépipède de façon à réaliser deux séries de gradins (8, 9) constitués de faces parallèles (10, 11, 12, ..., 14, 15, 16, ...) situées à des profondeurs croissantes sous la surface (2, 3) de la tôle (1), une première série de gradins (8) partant de la surface supérieure (2) de la tôle (1), une deuxième série de gradins (9) partant de la surface inférieure (3) de la tôle (1) et les deux séries de gradins (8, 9) étant disposés de telle sorte que chaque face (10, 11, 12, ...) d'une série de gradins (8) se trouve en vis-à-vis d'une face (14, 15, 16, ...) de l'autre série de gradins (9) et que la distance entre deux faces opposées ( 12, 15 ; 11, 16) soit la même pour toutes les faces.

7. Procédé selon la revendication 6, caractérisé en ce que les aires des faces des séries de gradins (8, 9) sont égales entre elles.

8. Procédé selon l'une quelconque des revendications 6 et 7, caractérisé en ce qu'on compte les cloques sur chaque face des séries de gradins (8, 9) pour obtenir une évaluation de la propreté inclusionnaire à différentes profondeurs sous la surface de la tôle (1).

## Patentansprüche

1. Verfahren zur Bewertung der Einschlußreinheit eines metallurgischen Produkts (1) durch Zählung von Einschlüssen im Innern des Produkts (1), dadurch gekennzeichnet, daß:
- man eine Probe (4) des metallurgischen Produkts (1) nimmt und man aus dieser Probe durch maschinelle Materialabhebung einen Probestab (7) derart herstellt, daß äußere Oberflächen (11, 12, 15, 16) des Probestabs von bestimmtem Flächeninhalt geschaffen werden, die sich in verschiedenen Tiefen unter den anfänglichen äußeren Oberflächen (2 ,3) des metallurgischen Produkts befinden (1),
- man den Probestab (7) in ein Bad taucht, das Wasserstoffionen und einen Wasserstoffrekombinationshemmer oder einen Wasserstoffabsorptionsbeschleuniger enthält, und zwar für eine Zeitdauer, die ausreicht, um ein signifikantes Eindringen von Wasserstoff in das Innere des Probestabs (7) zu erzielen,
- man die äußeren Oberflächen (10, 11, 12, 14, 15, 16) des Probestabs (7) untersucht, um für jede der äußeren Oberflächen (10, 11, 12, 14, 15, 16) die Zahl der Blasen zu ermitteln und zu zählen, die durch Anhebung einer Oberflächenschicht des Produkts (1) unter der Wirkung von unter Druck stehendem Wasserstoff gebildet wurden, welcher sich in Höhe jedes der unter der Oberflächenschicht nahe der äußeren Oberfläche befindlichen Einschlüsse gesammelt hat.

2. Verfahren nach Anspruch 1, dadurch gekennzeichnet, daß das Bad aus einer nicht oxydierenden sauren Lösung besteht.

3. Verfahren nach Anspruch 2, dadurch gekennzeichnet, daß das Bad aus Natriumchlorid-gesättigtem Wasser besteht, dessen pH-Wert durch Essigsäure auf 3 gehalten wird, das mit Schwefelwasserstoff H₂S gesättigt ist und dessen Temperatur etwa der Raumtemperatur entspricht.

4. Verfahren nach Anspruch 1, dadurch gekennzeichnet, daß der Probestab (7) während des Eintauchens in dem Bad auf ein kathodisches Potential gebracht wird.

5. Verfahren nach Anspruch 3, dadurch gekennzeichnet, daß man den Probestab (7) etwa vier Stunden in das Bad taucht.

6. Verfahren nach einem der Ansprüche 1 bis 5, dadurch gekennzeichnet, daß, wenn das Hüttenerzeugnis (1) eine Platte oder ein Blech ist, die Probe (4) ein Parallelepiped ist und der Probestab (7) erhalten wird, indem man dieses Parallelepiped maschinell so bearbeitet, daß zwei Reihen von Stufen (8, 9) entstehen, die von parallelen Flächen (10, 11, 12, ... 14, 15, 16, ...) gebildet werden, welche sich in zunehmenden Tiefen unter der Oberfläche (2, 3) des Bleches (1) befinden, eine erste Stufenreihe (8), die von der oberen Oberfläche (2) des Bleches (1) ausgeht, und eine zweite Stufenreihe (9), die von der unteren Oberfläche (3) des Bleches (1) ausgeht, wobei die beiden Stufenreihen (8, 9) derart angeordnet sind, daß jede Fläche (10, 11, 12, ...) einer Stufenreihe (8) einer Fläche (14, 15, 16, ...) der anderen Stufenreihe (9) gegenüberliegt und daß der Abstand zwischen zwei gegenüberliegenden Flächen (12, 15; 11, 16) für alle Flächen gleich ist.

7. Verfahren nach Anspruch 6, dadurch gekennzeichnet, daß die Flächeninhalte der Flächen der Stufenreihen (8, 9) untereinander gleich sind.

8. Verfahren nach einem der Ansprüche 6 und 7, dadurch gekennzeichnet, daß man die Blasen auf jeder Fläche der Stufenreihen (8, 9) zählt, um eine Bewertung der Einschlußreinheit in verschiedenen Tiefen unter der Oberfläche des Bleches (1) zu erhalten.

## Claims

1. Process for the evaluation of the inclusion cleanliness of a metallic product (1) by counting the inclusions inside the product (1), characterized in that:
- a sample (4) is taken of the metallic product (1) and a test piece (7) is machined from this sample, by the removal of material, so as to create external surfaces (11, 12, 15, 16) of the test piece, of a defined area, at different depths beneath the initial external surfaces (2, 3) of the metallic product (1),
- the test piece (7) is immersed in a bath containing hydrogen ions and a hydrogen recombination inhibitor or a hydrogen absorption promoter, for a sufficient period of time to obtain a significant penetration by the hydrogen inside the test piece (7),
- the external surfaces (10, 11, 12, 14, 15, 16) of the test piece (7) are examined in order to detect and count, for each of the external surfaces (10, 11, 12, 14, 15, 16) the number of blisters formed by the lifting of a surface layer of the product (1) under the influence of hydrogen under pressure which has accumulated in each of the inclusions situated beneath the surface layer, near to the external surface.

2. Process according to claim 1, characterized in that the bath is constituted by a non-oxidizing acid solution.

3. Process according to claim 2, characterized in that the bath is constituted by water saturated with sodium chloride, the pH of which is maintained at 3 using acetic acid, which is saturated with hydrogen sulphide H₂S and the temperature of which is close to ambient.

4. Process according to claim 1, characterized in that the test piece (7) is taken to a cathodic potential during immersion in the bath.

5. Process according to claim 3, characterized in that the test piece (7) is immersed in the bath for about four hours.

6. Process according to any one of claims 1 to 5, characterized in that when the iron or steel product (1) is a plate or a sheet, the sample (4) is a parallelepiped and the test piece (7) is obtained by machining said parallelepiped so as to produce two series of steps (8, 9) constituted by parallel faces (10, 11, 12, ..., 14, 15, 16, ...) situated at increasing depths from the surface (-2, 3) of the sheet (1), a first series of steps (8) starting from the upper surface (2) of the sheet (1), a second series of steps (9) starting from the lower surface (3) of the sheet (1) and the two series of steps (8, 9) being arranged such that each face (10, 11, 12, ...) of one series of steps (8) is opposite one face (14, 15, 16, ...) of the other series of steps (9) and that the distance between two opposing faces (12, 15; 11, 16) is the same for all of the faces.

7. Process according to claim 6, characterized in that the areas of the faces of the series of steps (8, 9) are all equal.

8. Process according to any one of claims 6 and 7, characterized in that the blisters are counted on each face of the series of steps (8, 9) in order to obtain an evaluation of the inclusion cleanliness at different depths beneath the surface of the sheet (1).
